# EUROPEAN PATENT APPLICATION

(11) **EP 3 922 684 A1**
(43) Date of publication of application: **15.12.2021**
(21) Application number: 21178863.3
(22) Date of filing: 10.06.2021
(51) Int. Cl.: C09D 5/14, A61L 2/232, A01N 25/34, B64D 11/00, E03C 1/00, E03D 11/00, E04B 1/00, E05B 1/00

(54) **ENHANCED MAINTENANCE ANTIVIRAL COATINGS FOR CABIN SURFACES**

(30) Priority: 10.06.2020 US 202063037267 P; 03.06.2021 US 202117338413
(71) Applicant: AMI Industries, Inc., Colorado Springs, CO 80916 (US)
(72) Inventor: GURVICH, Mark, East Hartford, 06108 (US)
(74) Representative: Dehns

(57) **Abstract**

A protective enhanced-maintenance antiviral coating (214) for interior components of a passenger cabin is disclosed. In embodiments, the enhanced-maintenance antiviral coating incorporates into the antiviral coating indicating agents, e.g., reflective or fluorescent compounds that combine with the antiviral coating without otherwise impeding its antiviral and/or antibacterial properties. The enhanced-maintenance antiviral coating is applied to external surfaces of the interior components of an aircraft cabin or other mobile platform especially prone to frequent physical contact by multiple individuals. The indicating agent reacts via reflectivity and/or fluorescence when ultraviolet (UV) band or otherwise specialized lighting is applied to the external surfaces. A lack of reflectivity or fluorescence may be interpreted as significant wear or damage to the antiviral coating, for which corrective actions may be taken.

## Description

### CROSS-REFERENCE TO RELATED APPLICATIONS

The present application is related to and claims the benefit of the earliest available effective filing dates from the following listed applications (the "Related Applications") (e.g., claims earliest available priority dates for other than provisional patent applications (e.g., under 35 USC § 120 as a continuation in part) or claims benefits under 35 USC § 119(e) for provisional patent applications, for any and all parent, grandparent, great-grandparent, etc. applications of the Related Applications).

### Related Applications:

United States Provisional Patent Application Serial No. 63/037,267 entitled ENHANCED MAINTENANCE ANTIVIRAL COATINGS FOR CABIN SURFACES, filed June 10, 2020;

Said United States Provisional Patent Application 63/037,267 is herein incorporated by reference in its entirety.

### TECHNICAL FIELD

The subject matter disclosed herein is directed generally to aircraft and vehicle interiors and more particularly for antiviral protection for the external surfaces of passenger cabin components.

### BACKGROUND

Aircraft passenger cabins may be prime spots or epicenters for the transmission of viruses, bacteria, or other pathogenic microorganisms from person to person, due to concentrating large groups of people in close proximity for long periods of time. Further, the passenger cabin may include multiple surfaces especially prone to physical contact by one or more passengers and thus particularly likely to serve as vectors for transmission via said personal contact. Antiviral and/or antibacterial coatings may provide resistance against viral transmission for these surfaces, but the said coatings may be relatively weak and liable to be eroded and/or compromised by sustained physical contact.

### SUMMARY

In a first aspect, an enhanced-maintenance protective antiviral coating for external surfaces of internal components within a passenger cabin or other like public space is disclosed. In embodiments, the enhanced-maintenance antiviral coating includes antiviral, antibacterial, and/or other components configured to destroy or reduce the effectiveness of pathogens, such as viruses, bacteria, and other like pathogenic microorganisms deposited upon a treated external surface. The coating includes indicating agents incorporated therein; when specialized light (e.g., incorporating specially selected reactive components) is applied to the external surface, the indicating agents reveal an absence or thickness change of the antiviral compound (which may be sufficiently severe to warrant replacement, repair, or other corrective action).

In some embodiments, the indicating agents include fluorescing agents capable of absorbing the specialized light.

In some embodiments, the indicating agents include reflective agents capable of partially or fully reflecting the specialized light.

In some embodiments, the indicating agents include reactive particles suspended within the antiviral compound.

In some embodiments, the specialized light induces fluorescence in the indicating agents.

In some embodiments, the specialized light includes visible light and/or ultraviolet (UV) spectral components.

In some embodiments, the enhanced-maintenance antiviral compound is a multilayer compound, wherein multiple types of indicating agents may be incorporated into the antiviral compound and applied to the external surface in a sequence of layers, such that the exact layer revealed to the specialized light (and the exact indicating agents incorporated thereinto) may reveal precise information about thickness changes to the antiviral coating.

In some embodiments, the varied layers of the enhanced-maintenance antiviral compound may react by fluorescing in specific colors to indicate the depth of wear to, or the remaining thickness of, the antiviral coating.

In some embodiments, the indicating agents are incorporated into a parasitic layer disposed beneath the antiviral coating, indicating the wearing away of the antiviral coating over a region or area by their fluorescence or reflectivity.

In some embodiments, the public space is a passenger cabin configured for transport of a group of passengers.

In some embodiments, the passenger cabin is disposed within an aircraft, a ground-based vehicle, or a water-based vehicle.

In some embodiments, the public space includes a classroom, theater, auditorium, office space, or other indoor interior space.

In a further aspect, a method for maintaining a public interior area comprising multiple surfaces associated with frequent or continual physical contact by multiple individuals is disclosed. In embodiments, the method includes providing an enhanced-maintenance antiviral coating by adding fluorescent, reflective, and/or reactive dyes or other indicating agents to an antiviral or antimicrobial coating. The method includes applying the enhanced-maintenance antiviral coating to the external surfaces associated with frequent or continual physical contact, e.g., toilet lids, walls and panels, faucets, light switches, door/panel handles, buttons, seating components. The method includes applying specialized lighting to the coated surfaces to check the status of any antiviral or antimicrobial coatings applied thereto. The method includes detecting changes in fluorescence or reflectivity indicative of significant damage or wear to the coated external surfaces. The method includes taking corrective actions with respect to any detected areas of damage or wear, e.g., reapplying the enhanced-maintenance antiviral coating to the areas of damage or wear, reapplying the enhanced-maintenance antiviral coating uniformly or non-uniformly to the damaged or worn external surface, or replacing the damaged or worn external surface or its embodying component.

This Summary is provided solely as an introduction to subject matter that is fully described in the Detailed Description and Drawings. The Summary should not be considered to describe essential features nor be used to determine the scope of the Claims. Moreover, it is to be understood that both the foregoing Summary and the following Detailed Description are example and explanatory only and are not necessarily restrictive of the subject matter claimed.

### BRIEF DESCRIPTION OF THE DRAWINGS

The detailed description is described with reference to the accompanying figures. The use of the same reference numbers in different instances in the description and the figures may indicate similar or identical items. Various embodiments or examples ("examples") of the present disclosure are disclosed in the following detailed description and the accompanying drawings. The drawings are not necessarily to scale. In general, operations of disclosed processes may be performed in an arbitrary order, unless otherwise provided in the claims. In the drawings:
FIG. 1 is an isometric overhead view of an aircraft lavatory incorporating enhanced-maintenance antiviral coatings according to example embodiments of this disclosure;
FIG. 2 is an isometric profile view of an aircraft seating environment incorporating enhanced-maintenance antiviral coatings according to example embodiments of this disclosure;
FIG. 3A is an overhead view illustrating the enhanced-maintenance antiviral coating of FIGS. 1 and 2;
FIG. 3B is a cross sectional through-thickness diagrammatic illustration of the enhanced-maintenance antiviral coating of FIG. 3A;
FIG. 3C is a cross sectional through-thickness diagrammatic illustration of the enhanced-maintenance antiviral coating of FIG. 3A;
FIG. 4 is a flow diagram illustrating a method for service enhancement according to example embodiments of this disclosure;
FIG. 5A is a cross sectional through-thickness diagrammatic illustration of the enhanced-maintenance antiviral coating of FIG. 1;
FIG. 5B is a cross-sectional through-thickness diagrammatic illustration of the enhanced-maintenance antiviral coating of FIG. 1;
FIG. 5C is a cross-sectional through-thickness diagrammatic illustration of the enhanced-maintenance antiviral coating of FIG. 1.
FIG. 6 is a cross-sectional through-thickness diagrammatic illustration of an external surface with an enhanced-maintenance antiviral coating according to example embodiments of this disclosure;
and FIG. 7 is a cross-sectional through-thickness diagrammatic illustration of an external surface with an enhanced-maintenance antiviral coating according to example embodiments of this disclosure.

### DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment disclosed herein. The appearances of the phrase "in some embodiments" in various places in the specification are not necessarily all referring to the same embodiment, and embodiments may include one or more of the features expressly described or inherently present herein, or any combination or sub-combination of two or more such features, along with any other features which may not necessarily be expressly described or inherently present in the instant disclosure.

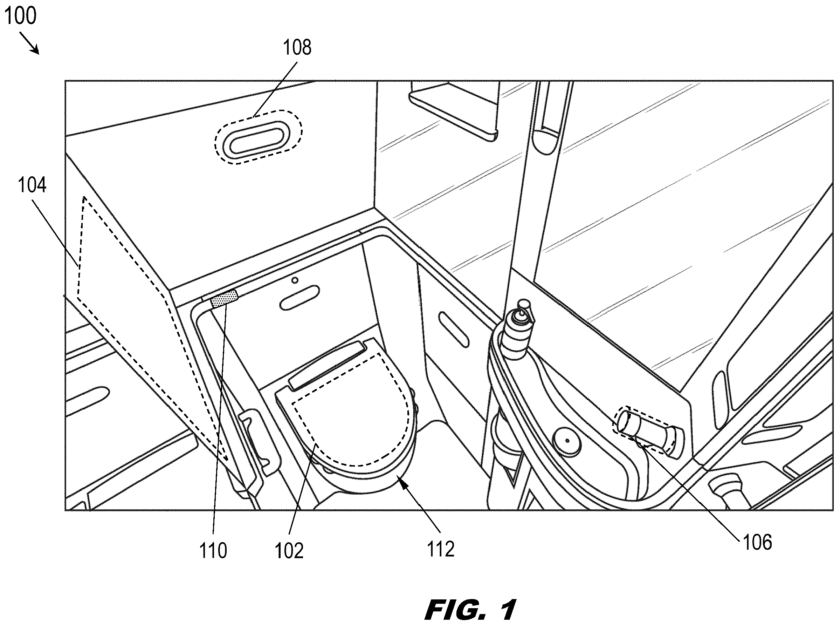

Referring to FIG. 1, an aircraft lavatory 100 is disclosed.

The aircraft lavatory 100, and other interior areas of an aircraft cabin, may be areas of especially high potential danger for transmission of viruses (e.g., COVID-19 or other types of respiratory viruses), bacteria, and other pathogenic microorganisms from one person to another. While the inventive concepts disclosed herein may be implemented within any mobile platform or public interior space, commercial aircraft provide a particularly strong opportunity for application. Aircraft passenger cabins congregate large groups of people in close proximity under recirculating air systems for longer periods of time than do most ground-based vehicles. Within any one flight, individual passengers and/or crewmembers may originate from dozens or even hundreds of locations around the world, which may include epidemiological epicenters. The aircraft lavatory 100 may be occupied and used by several dozen individuals over the course of a flight and may include numerous objects and/or surfaces especially likely to experience physical contact from multiple passengers, e.g., toilet lids 102, interior walls and panels 104, faucets 106, door/panel handles 108, or buttons 110 (e.g., for flushing the toilet 112).

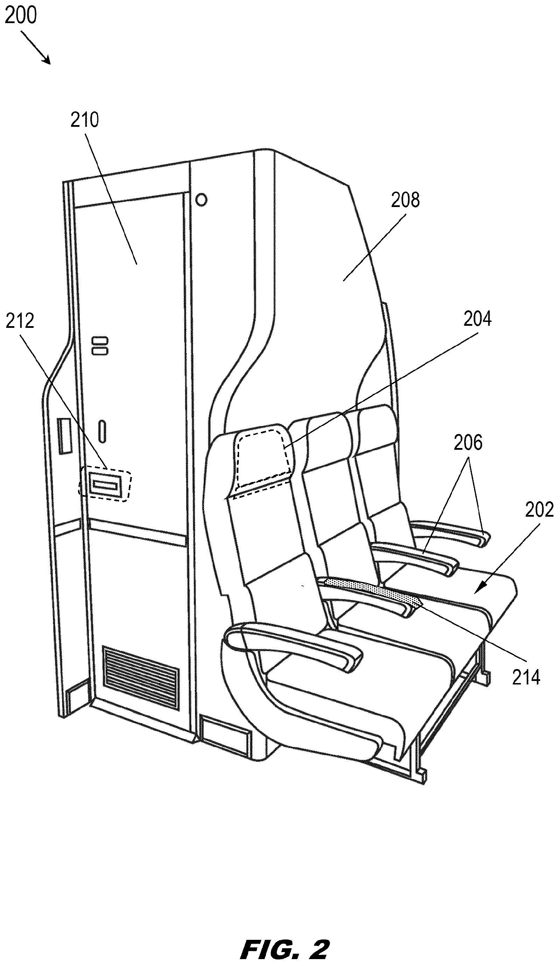

Referring also to FIG. 2, an aircraft seating environment 200 is disclosed. Similarly to FIG. 1, in embodiments the aircraft seating environment 200 may include individual passenger seats 202, headrests 204, armrests 206 (which may be shared between adjacent seats), and aircraft monuments 208 including, e.g., an access door 210 (with access handle/latch 212) for the aircraft lavatory 100 of FIG. 1. These locations may be especially susceptible to physical contact by one or more passengers throughout the course of a flight (as opposed to, e.g., cabin ceilings) and may thus be prime candidates for viral or bacterial transmission via physical contact.

In embodiments, the toilet lids 102, interior walls and panels 104, faucets 106, headrests 204, armrests 206, access handle/latch 212, and/or other external surfaces particularly prone to physical contact may be partially or fully coated with an enhanced-maintenance antiviral coating (214) infused with indicative agents to facilitate service and replacement. For example, the enhanced-maintenance antiviral coating 214 may indicate to the cabin crew and/or maintenance team if, and precisely where, the antiviral coating has eroded or otherwise worn from the surface onto which it has been applied (and thus if, and where, the antiviral coating should be serviced (e.g., repaired, reapplied, replaced). In some embodiments, the enhanced-maintenance antiviral coating 214 may be applied to the external surfaces of interior components within the passenger cabins of other passenger and transit vehicles (e.g., railway cars, buses, trolleys, ferries, passenger ships, cable cars, funiculars) or within indoor public spaces configured for the concentration of populations in close proximity for extended periods, e.g., classrooms, office spaces, theaters and auditoriums.

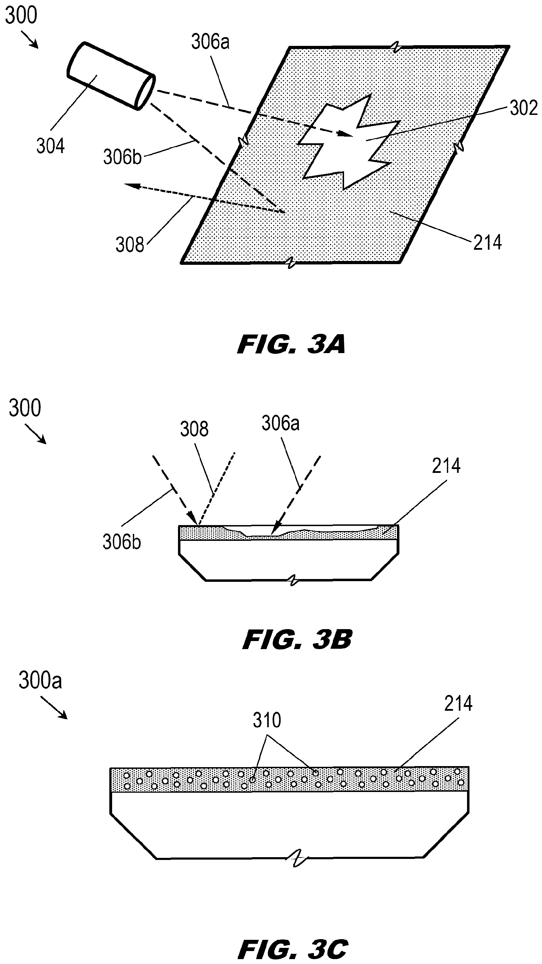

Referring to FIGS. 3A and 3B, in embodiments an external surface 300 e.g., of a toilet lid 102, interior wall or panel 104, faucet 106, headrest 204, armrest 206, access handle/latch 212, and/or other interior component within the passenger cabin) may have an enhanced-maintenance antiviral coating 214 applied thereto. Over time, especially after sustained physical contact, the enhanced-maintenance antiviral coating 214 may experience wear (302). For example, the disinfectant components of the enhanced-maintenance antiviral coating 214 may lose potency overtime. More commonly, however, the enhanced-maintenance antiviral coating 214 may be reduced in thickness as the coating is abraded or erodes from the external surface 300.

In embodiments, the enhanced-maintenance antiviral coating 214 may incorporate one or more indicating agents combined into, or commingled with, the disinfectant components of the antiviral coating. For example, the enhanced-maintenance antiviral coating 214 may include fluorescent dyes or agents mixed by any appropriate processes into the antiviral coating in such a way as not to impede its antiviral properties. The enhanced-maintenance antiviral coating 214 may appear invisible or otherwise normal under standard interior lighting (e.g., indistinguishable from the untreated external surface). However, the cabin crew or maintenance team may quickly inspect the external surface 300 and enhanced-maintenance antiviral coating 214 by illuminating the antiviral coating with a dedicated light source 304.

In embodiments, the dedicated light source 304 may emit specialized light (306a-b) partially or fully within the ultraviolet (UV) band (e.g., in addition to visible light components) or incorporating other specially selected spectral components to which the indicating agents are reactive, such that the indicating agents react to the emitted light and indicate a status (e.g., a presence, an absence, a thickness change) of the enhanced-maintenance antiviral coating 214. The presence, absence, or thickness change of the enhanced-maintenance antiviral coating 214 may be full or partial. For example, the specialized light 306a-b may induce fluorescence in the indicating agents within the enhanced-maintenance antiviral coating 214, which may absorb the specialized light 306b, re-emitting the absorbed light (308) at a slightly different wavelength. The crew or maintenance team member, scanning the external surface 300 with the dedicated light source 304, may view the re-emitted light/fluorescence 308 e.g., in a specific color, or otherwise as an indication that the enhanced-maintenance antiviral coating 214 remains intact (e.g., fully intact, or sufficiently intact). In some embodiments, the indicating agents may be partially or fully reflective in nature.

In embodiments, when the dedicated light source 304 is directed toward a portion of wear (302) the absence of enhanced-maintenance antiviral coating 214 (and thus the absence of indicating agents) will be reflected in an appearance, under specialized light 306a, different from the re-emitted light/fluorescence 308. For example, the lack of fluorescence or reflectivity may indicate damage to, or erosion of, the enhanced-maintenance antiviral coating 214 sufficient to warrant service or replacement.

Referring now to FIG. 3C, in embodiments the external surface 300a may be implemented and may function similarly to the external surface 300 of FIGS. 3A and 3B, except that within the enhanced-maintenance antiviral coating 214, the indicating agents (310) may appear (e.g., under the specialized light 306a-b, FIGS. 3A-B) as particles suspended in the antiviral coating (as opposed to, e.g., seamless incorporation with the enhanced-maintenance antiviral coating 214).

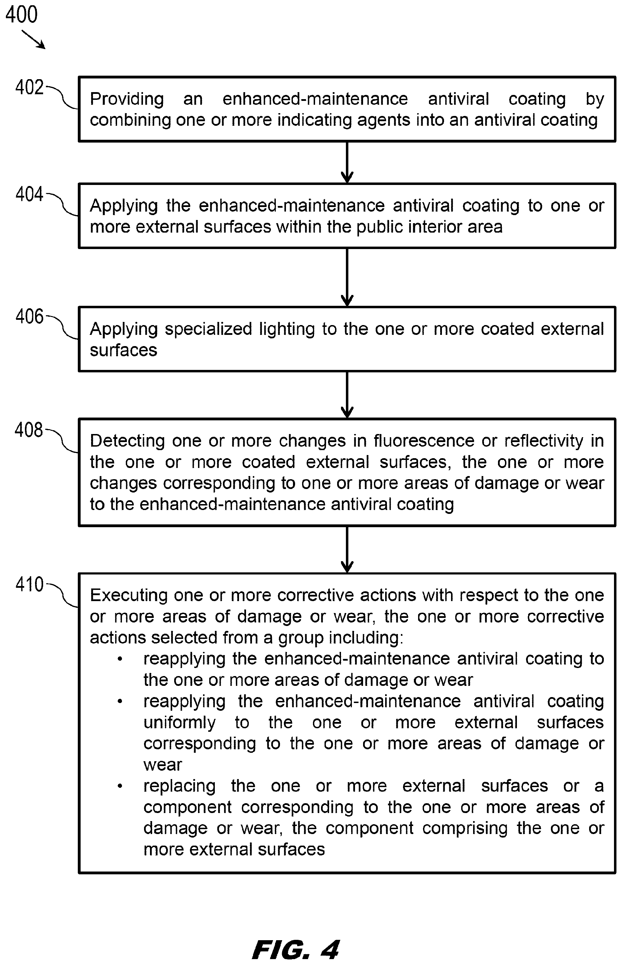

Referring now to FIG. 4, a method 400 of service is disclosed. The method 400 may be implemented within the aircraft lavatory 100 of FIG. 1, the aircraft seating environment 200 of FIG. 2, or any other public interior area of a vehicle or mobile platform incorporating external surfaces of interior components associated with frequent or continual physical contact by multiple individuals.

At a step 402, cabin crew (or, e.g., maintenance personnel or interior suppliers) may combine one or more indicating agents (e.g., fluorescent, reflective, reactive) with or into an antiviral or/and antimicrobial coating.

At a step 404, the cabin crew may apply the enhanced-maintenance antiviral coating to one or more external surfaces of interior components (e.g., as disclosed above in FIGS. 1 and 2 and accompanying text).

At a step 406, the cabin crew may apply ultraviolet or otherwise specialized lighting to the applied enhanced-maintenance antiviral coating, checking for changes in its fluorescence and/or reflectivity.

At a step 408, the observing cabin crew may recognize areas of reduced or eliminated fluorescence and/or reflectivity as areas of damage or wear to the enhanced-maintenance antiviral coating.

At a step 410, the observing cabin crew may take a variety of corrective actions in response to detected damage or wear: reapplying the enhanced-maintenance antiviral coating locally to damaged or worn areas, reapplying the enhanced-maintenance antiviral coating (e.g., uniformly or non-uniformly) to the entire external surface, or replacing the affected external surface or internal component.

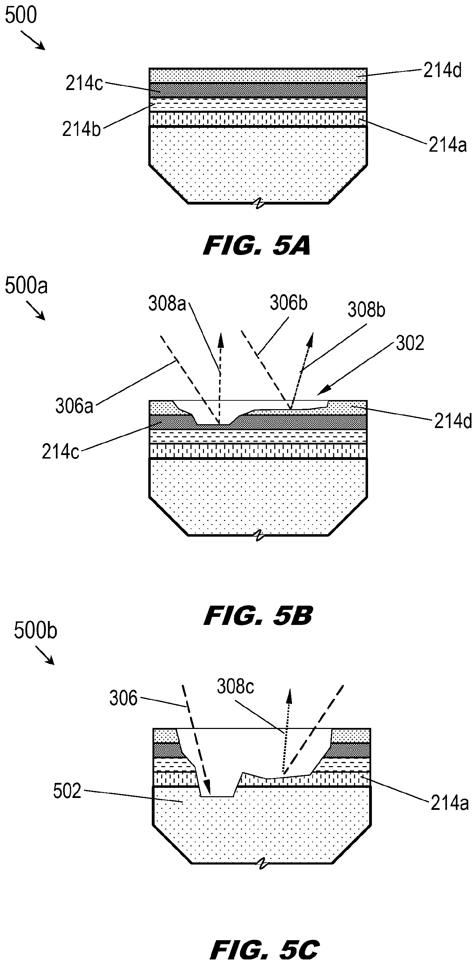

Referring to FIG. 5A, in embodiments the external surface 500 may be implemented and may function similarly to the external surface 300 of FIG. 3A, except that the external surface 500 may have applied thereto a series of layered enhanced-maintenance antiviral coatings 214a-d.

In embodiments, the series of layered enhanced-maintenance antiviral coatings 214a-d may be applied in succession over the external surface 500. For example, the enhanced-maintenance antiviral coating 214a may be fashioned by adding a first indicating agent to the antiviral coating, such that the antiviral coating has a distinct appearance (e.g., a generally red color). Similarly, the enhanced-maintenance antiviral coating 214b may be fashioned by adding a second indicating agent to the antiviral coating, such that the antiviral coating has an appearance distinct from the enhanced-maintenance antiviral coating 214a (e.g., a yellow color).

In embodiments, the enhanced-maintenance antiviral coating 214b may be applied over the enhanced-maintenance antiviral coating 214a, and the enhanced-maintenance antiviral coatings 214c-d (having, e.g., generally green and blue appearances, respectively).

Referring also to FIGS. 5B and 5C, in embodiments the external surfaces 500a and 500b may be implemented and may function similarly to the external surface 500 of FIG. 5A, except that the external surfaces 500a-b may sustain wear (302) or damage. In embodiments, when the specialized light 306a-b is applied to the external surfaces 500a-b, the wear 302 or damage may be revealed more precisely by the reflectivity or fluorescence 308a-c emitted thereby. For example, referring in particular to FIG. 5B, blue and green fluorescence 308a-b may be respectively associated with minimal or minor damage or wear respectively revealing the enhanced-maintenance antiviral coatings 214d and 214c. Similarly, referring in particular to FIG. 5C, significant damage or wear revealing the enhanced-maintenance antiviral coating 214a may be associated with red fluorescence 308c, and severe or total damage or wear may erode or abrade the enhanced-maintenance antiviral coating 214a-d entirely, revealing the original component surface 502 (e.g., via the absence of fluorescence or reflectivity) when the specialized light 306 is applied to the external surface.

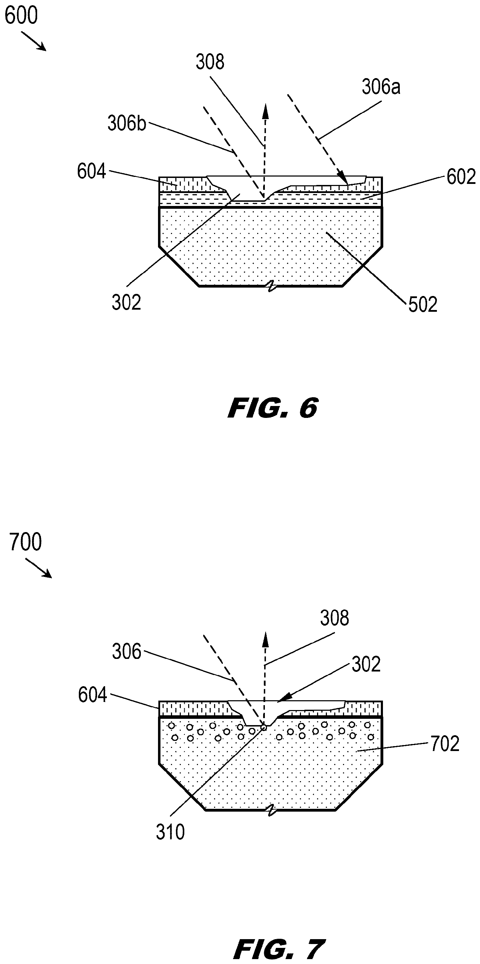

Referring to FIG. 6, the external surface 600 is disclosed.

In embodiments, the external surface 600 may incorporate a parasitic layer 602 under an antiviral coating 604. For example, the parasitic layer 602 may incorporate fluorescent or reflective indicators in one or more layers adjacent to the original component surface 502. The external surface 600 may otherwise be implemented similarly to the external surfaces 300, 500 of FIGS. 3A, 3B, and 5A, except that when specialized light 306a is applied thereto, the lack of fluorescence and/or reflectivity may be interpreted as a lack of damage or wear. Similarly, if damage or wear (302) to the antiviral coating 604 is present, only then may the parasitic layer 602 exhibit fluorescence or reflectivity (308) when specialized light 306b is applied thereto.

Referring to FIG. 7, in embodiments the external surface 700 may be implemented and may function similarly to the external surface 600 of FIG. 6, except that the external surface 700 (e.g., of an interior component as described above) may embed or otherwise incorporate indicating agents 310 directly into a surface layer 702. For example, if damage or wear (302) occurs to the antiviral coating 604, the surface layer 702 below may exhibit fluorescence or reflectivity (308) when specialized light 306 is applied thereto.

## Claims

1. A protective antiviral coating (214) for interior components of a public space, comprising:
at least one antiviral compound applicable to an external surface (300) within a public space and configured to destroy one or more pathogens deposited on the external surface;
and
at least one indicating agent combined with the at least one antiviral compound, the indicating agent configured to indicate a status of the antiviral compound by reacting to a specialized light applied to the external surface.

2. The protective antiviral coating of claim 1, wherein the status is selected from a presence of the antiviral compound, an absence of the antiviral compound, or a change of the antiviral compound.

3. The protective antiviral coating of claims 1 or 2, wherein the at least one indicating agent includes at least one fluorescing agent configured to absorb the specialized light.

4. The protective antiviral coating of any preceding claim, wherein the at least one indicating agent includes at least one reflective agent configured to reflect the specialized light.

5. The protective antiviral coating of any preceding claim, wherein the at least one indicating agent includes one or more reactive particles suspended within the antiviral compound.

6. The protective antiviral coating of any preceding claim, wherein the specialized light is configured to induce fluorescence in the at least one indicating agent.

7. The protective antiviral coating of claim 6, wherein the specialized light includes at least one of visible light or ultraviolet (UV) light.

8. The protective antiviral coating of any preceding claim, wherein the at least one indicating agent comprises:
at least one first indicating agent configured to combine with the antiviral compound in an outermost layer, the first indicating agent configured to indicate a status of the outermost layer via a first reaction to the specialized light;
and
at least one second indicating agent configured to combine with the antiviral compound in at least one secondary layer beneath the outermost layer, the second indicating agent configured to indicate a status of each corresponding secondary layer via at least one second reaction to the specialized light.

9. The protective antiviral coating of claim 8, wherein:
the first reaction is associated with a first color;
and
the at least one second reaction is associated with at least one second color.

10. The protective antiviral coating of any of claims 1 through 7, wherein the at least one indicating agent is configured to:
combine with the at least one antiviral compound in at least one layer between the antiviral compound and the external surface;
and
indicate a status of the antiviral compound by reacting to the specialized light.

11. The protective antiviral coating of any preceding claim, wherein the public space includes a cabin within a vehicle, the vehicle configured for transport of one or more passengers.

12. The protective antiviral coating of claim 11, wherein the vehicle is selected from an aircraft, a ground-based vehicle, or a water-based vehicle.

13. The protective antiviral coating of any of claims 1 through 10, wherein the public space includes an interior space within a building.
